# EUROPEAN PATENT SPECIFICATION

(11) **EP 3 902 463 B1**
(45) Date of publication and mention of the grant of the patent: **12.02.2025**
(21) Application number: 19845871.3
(22) Date of filing: 26.12.2019
(51) Int. Cl.: A61B 5/0215, A61B 5/07, A61B 5/00

(54) **SENSOR DELIVERY SYSTEM**
SENSORZUFÜHRUNGSSYSTEM
SYSTÈME DE POSE DE CAPTEUR

(30) Priority: 26.12.2018 US 201862784887 P; 09.01.2019 US 201916243183
(43) Date of publication of application: 03.11.2021
(73) Proprietor: Endotronix, Inc., Lisle, IL 60532 (US)
(72) Inventor: CHRONOS, Nicholas, Lisle, IL 60532 (US); NAGY, Michael, Lisle, IL 60532 (US); ROWLAND, Harry, Lisle, IL 60532 (US); PANIAN, Tyler, Lisle, IL 60532 (US); WILSCHKE, Thomas, Lisle, IL 60532 (US); ROYER, Trace, Lisle, IL 60532 (US); COYLE, James, Lisle, IL 60532 (US); MAHR, David, Lisle, IL 60532 (US); FOROUZAN, Omid, Lisle, IL 60532 (US); POFF DVM, Brad, Lisle, IL 60532 (US)
(74) Representative: Müller-Boré & Partner Patentanwälte PartG mbB
(86) International application number: PCT/US2019/068551
(87) International publication number: WO 2020/139928

(56) References cited:
- US-A- 5 411 551
- US-A1- 2003 125 790
- US-A1- 2006 135 963
- US-A1- 2008 176 271
- US-A1- 2015 208 929
- US-A1- 2016 324 443

## Description

### CROSS-REFERENCE TO RELATED APPLICATIONS

This application claims priority to U.S. Provisional Patent App. No. 62/784,887 entitled "SENSOR DELIVERY SYSTEM AND METHOD" and filed December 26, 2018. This application is a continuation-in-part of U.S. Patent App. No. 16/243183 entitled "PRESSURE SENSOR, ANCHOR, DELIVERY SYSTEM AND METHOD and filed January 9, 2019 which claims priority to U.S. Patent No. 10,206,592 entitled "PRESSURE SENSOR, ANCHOR, DELIVERY SYSTEM AND METHOD and filed March 16, 2015 which claims priority to PCT Patent App. No. PCT/US2013/059769 entitled "PRESSURE SENSOR, ANCHOR, DELIVERY SYSTEM AND METHOD and filed on September 13, 2013, that claims priority to U.S. Provisional Patent App. No. 61/701,058 entitled "PRESSURE SENSOR, ANCHOR, DELIVERY SYSTEM AND METHOD" and filed on September 14, 2012, and further claims priority to PCT Patent App. No. PCT/US2011/045583 entitled "PRESSURE SENSOR, CENTERING ANCHOR, DELIVERY SYSTEM AND METHOD" and filed on July 27, 2011. This application is also a continuation-in-part of U.S. Patent App. No. 15/213,712 entitled "PRESSURE SENSING IMPLANT," filed on July 19, 2016 which is a continuation-in-part of U.S. Patent Application No. 14/777,654 entitled "PRESSURE SENSING IMPLANT" filed on September 16, 2015.

### FIELD OF INVENTION

This application relates to a medical implantable devices, positioning and anchoring mechanisms, delivery systems and more particularly to a method for delivering and positioning medical implantable devices into the human body.

### BACKGROUND

Delivery systems and positioning and anchoring devices are currently being used in medical procedures to guide and position devices from a remote site to a target site within a body. Catheter-based delivery systems are generally used to guide and position invasive implantables, such as pressure sensors, within the cardiovascular system of a patient. There exist various commercial implantable pressure sensors that are permanently implanted within the cardiovascular system using catheter-based delivery systems. One example is the CardioMEMS device by Abbott Labs, Inc. References: "CardioMEMS HF System, PA Sensor and Delivery System", St Jude Medical Publication US-2000054 B EN (06/14); Shavelle, D, MD; Jermyn, R, MD; "The CardioMEMS Heart Failure Sensor: A Procedural Guide for Implanting Physicians", Journal of Invasive Cardiology 2016; 28(7):273-279*.;* "CardioMEMS HF System, PA Sensor and Delivery System, Model CM2000, User's Manual", CardioMEMS publication LA-400275-03, Art 60056412, May 2014; "CardioMEMS Implant Procedure and Patient Training Video" St Jude Medical video SJM-MEM-0215-0085a. 2015.

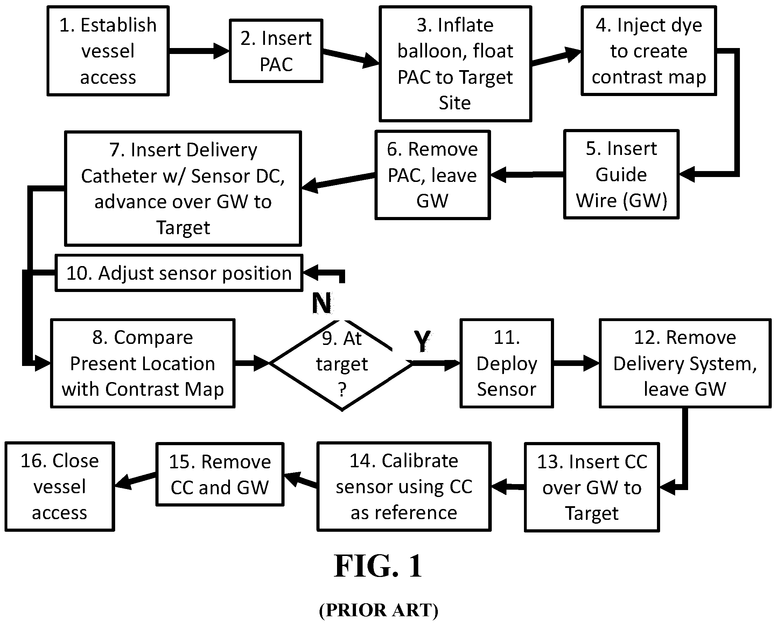

Figure 1 illustrates a schematic diagram of a prior art catheter-based delivery system disclosed by the described publications that describes a method for implanting a pressure sensor implant within the pulmonary artery of a patient using a pulmonary artery catheter ("PAC"). Here. the PAC includes a catheter having an inflatable balloon along a distal end of an elongated tube structure. The PAC is configured to provide fluid communication from a target site to the proximal end of the catheter outside the body. The PAC allows for pressure measurements as well as drug injections. It may also have other features, such as ability to measure cardiac output using the well-known thermodilution method. In a known embodiment, the target site includes the pulmonary artery but may be any other location within a patient's anatomy, preferably within the cardiovascular system.

The steps disclosed by this procedure include the following as illustrated by Figure 1. Venous access may be established at an access location along the patient's anatomy by puncturing a vein percutaneously (1). This may also include inserting an "introducer" at the access location to support the insertion of various catheters. The femoral vein is a typical access location; jugular, brachial, and subclavian vein are also known access locations. The distal end of a first pulmonary artery catheter ("PAC") is then inserted at the access location (2). A balloon may be inflated along a distal portion or distal top of the PAC within the vascularity of the patient (3). The inflated balloon may be used to free up blocked vessels or temporarily block blood flow to a certain branch. It may also be used to float the PAC along with the bloodstream towards the target anatomy. The target site may be anything in the path of venous flow, including any of the veins, the heart, the pulmonary artery, or even inside the gastrointestinal (GI) tract. The PAC may also be configured to take pressure readings or measure output (via thermodilution) along its journey through the anatomy up to and including the target site. Thremodilution is a method of measuring blood flow based on the premise that when an indicator substance is added to circulating blood, the rate of blood flow is inversely proportional to the indicator concentration change over time. The measurements taken of the target site, for example, could include but is not limited to the right atrial pressure, right ventricular pressure, pulmonary artery pressure, and pulmonary artery wedge pressure.

Contrast dye may be injected through a port in the PAC to assist with imaging and examining the anatomy with a fluoroscope (4). The contrast dye may be injected into the anatomy by introducing the contrast dye through the proximal end of the PAC outside the body, to be dispensed near the distal end of the PAC within the body. Imaging display may then clearly illustrate the vasculature with the contrast dye. This process is called angiography and the image you get from it is an angiogram. Prior art systems utilize static photos / freeze-frames of the angiogram to assist with positioning the sensor or implant with a second catheter as described below.

Preferably, during this procedure, the distal end of the PAC may be located at or near the target site and a guide wire (GW) may be inserted or positioned within a lumen of the PAC (5). The guide wire (GW) may slide or otherwise be withdrawn from a lumen within the PAC to be positioned at or adjacent the target site (6). The PAC may be removed allowing the guide wire (GW) to remain positioned therein. The guide wire (GW) may remain located within the vasculature between the access location and the target site to guide other catheters in and out to the target site (7).

A delivery catheter (DC) including a sensor or implant attached at or near a distal end thereto may be inserted through the access location of the patient with the assistance of the guide wire (GW) to positon the sensor or implant towards the target site. The fluoroscope may visually assist the medical practitioner to monitor the anatomy as the sensor or implant is positioned at or near the target site. The sensor may include fluoro markers to allow the visual assistance of the sensor or implant on the fluoroscope as well as guide wire (GW). However, at this point in the procedure of known delivery systems, contrast dye is not available and live images of the position of the sensor/implant along the DC relative to the vasculature is therefore not available (8). The medical practitioner must utilized the angiogram image acquired when contrast dye was introduced by the PAC moments ago to compare the relative position of the DC and sensor relative to anatomical markers (e.g., ribs, pacemaker, spine). This comparison of step (8) allows the medical practitioner to estimate sizes and locations using known features available from a static image of the angiogram in the estimation of placing the sensor or implant at the target site in the anatomy.

This requires the medical practitioner in control of the delivery catheter to stop and hold its position as the comparison occurs between the live fluoroscopic image (of the sensor) with the static angiogram "map" made moments ago. Steps (9) and (10) reflect the continual iterative process of moving or adjusting the delivery catheter and comparing the angiogram "map" with the live fluoro image until the medical practitioner in control of the deliver catheter estimates that the sensor or implant is positioned at the "estimated" target site. Once reached, the sensor or implant is deployed at the estimated target site (11). This may occur by triggering a mechanism at the proximal end of the delivery catheter that actuates release wires attached to the sensor or implant to release the senor or implant therefrom. The sensor is now deployed and the delivery catheter is removed from the patient (12) as the guide wire remains therein.

A second calibration catheter ("CC"), similar in type to the first pulmonary artery catheter PAC, may be inserted at access location along the guide wire (13). The second calibration catheter CC may include a fluid lumen therein but may not include a distally positioned balloon. Notably, each of these types of catheters are designed for a single use and may be costly. The fluid lumen may extend along the length of the second catheter CC and couple the proximal end to a second sensor. The second sensor may be an off-the-shelf one-use pressure sensor or transducer device that is used to obtain a reference reading (14). The second sensor may be placed at or near the target site that includes the sensor or implant for taking the reference reading. That reference reading may be used to adjust, correct, or otherwise calibrate the sensor or implant as it is placed at the target site. This is "in situ calibration" of the implanted sensor may be performed mathematically in software. The second catheter CC and guide wire GW may be removed (15) and the access location or vessel site may be closed (16).

Even though such implant procedures are minimally invasive and have a good safety record, any surgical procedure causes risk to the patient. There is a desire to minimize the time a patient is under anesthesia and exposed to risk of infection, also to minimize procedure time, use of medical resources, like a catheterization lab and implanting surgical teams. There is also a desire to reduce cost of healthcare by minimizing the amount of disposable and non-disposable equipment required for a given procedure. There is a need to improve the simplicity and efficiency of similar systems and methods for convenience of the medical practitioners and to enhance patient safety.

US2015/208929 A1 describes an implant delivery system including an implant, such as a wireless sensor, a first sheath, and a second sheath. The sheaths extend from a proximal end of the implant delivery system, and at least said first sheath extends to a distal end of said implant delivery system. The first sheath is positioned at least partially within said second sheath. The implant is connected to an exterior surface of the first sheath and positioned near an end of the second sheath. The first sheath and said second sheath are movable with respect to one another to deploy said implant to a desired location.

US 2016/324443 A1 describes an implant and method of making an implant. The implant having a housing that defines a cavity. The housing includes a sensor comprising a base attached to a diaphragm wherein said base may be positioned within said cavity. The sensor may be a capacitive pressure sensor. The diaphragm may be connected to the housing to hermetically seal said housing. The sensor may include electrical contacts positioned on the diaphragm. The base may define a capacitive gap and a vent wherein the electrodes may be positioned within said capacitive gap such that at least a portion of the electrical contacts extend through the vent. The implant may include a coil in electric communication with the sensor, said coil may be positioned within said housing. A printed circuit board having at least one component may be attached to the floating base.

US 2006/135963 A1 describes an expandable transluminal sheath, for introduction into the body while in a first, low cross-sectional area configuration, and subsequent expansion of at least a part of the distal end of the sheath to a second, enlarged cross-sectional configuration. The sheath is configured for use in the gastrointestinal system and has utility in the performance of endoscopic retrograde cholangiopancreatography (ERCP). The distal end of the sheath is maintained in the first, low cross-sectional configuration and expanded using a radial dilatation device. In an exemplary application, the sheath is utilized to provide access for a diagnostic or therapeutic procedure such as gallstone or pancreatic stone removal.

### SUMMARY

The underlying technical problem is solved by the implant delivery system according to independent claim 1. Additional embodiments are defined in the dependent claims. At least one fluid port may be positioned along a proximal end of said first sheath or second sheath, said fluid port fluidly coupled to a lumen extending down the length of said first sheath or second sheath to allow fluid flow through said lumen. Fluid may be injected through said port and may includes one of: a drug; a fluid used to enhance anatomical imaging; fluoroscopic contrast dye; barium; a radioactive material; blood; plasma; saline solution; a blood component; a particle suspension; a nano-device; and a nanomaterial. The at least one fluid port may further be configured to operatively couple to a device located outside of said body wherein said device is a pressure transducer, configured to measure a fluid pressure at the distal end of said first or said second sheath. Said measurement of fluid pressure at the distal end of said first or second sheath may be used to calibrate or assess the accuracy of said implant.

At least one marker may be placed on the delivery system that is configured to be visible with a fluoroscope. Said marker may include a radio opaque material positioned on at least one of: a distal tip of said first sheath; the distal portion of said second sheath; a portion of said implant; and as a plurality of lines spaced along a portion of said first or said second sheath. The marker may be attached to at least one anchor on the implant. A plurality of markers is positioned along said implant in an asymmetric pattern, said pattern is configured to facilitate determination of implant orientation when viewed on a fluoroscope. Said asymmetric pattern comprises markers at three of the four corners of a two-dimensional rectangle when viewed normal to the plane of said rectangle on a fluoroscope.

Said fluid port may be further configured to allow removal of fluid from said body. A balloon member may be positioned along the delivery system, wherein said balloon member is configured to be inflated to guide said deliver system to a target site. Said balloon member may be configured to facilitate a wedge pressure measurement. Said balloon member may be configured to limit blood flow in a vessel to facilitate implant deployment or retraction. Said balloon member may be configured to hold said first sheath in place with respect to a blood vessel while said implant is released from said first sheath at said target site. A temperature sensor may be placed on said distal end to facilitate measurement of flow rate by thermodilution, wherein said flow rate measurement is configured to determine a cardiac output.

Said second sheath may be configured to allow insertion of a catheter device, wherein said catheter device includes at least one of: a camera, a pressure sensing catheter, a stent placement device, a valve placement device, a microphone, an ablation device, a balloon device, a Swan Ganz catheter, an electrical stimulation device, an ultrasound device, a drug delivery device, a catheter for gripping implanted devices, a catheter for readjusting the position of implanted devices, a catheter for removing implanted devices. Said second sheath may be configured to allow insertion of a catheter device configured to selectively attach to said implant and move the implant proximally when retracted, wherein at least one anchor of said implant is collapsible, and wherein said anchor of said implant is configured to be placed in a collapsed state when said catheter device moves the implant proximally into said second sheath. Said second sheath may be further configured to allow said first sheath to be retracted into said second sheath while said implant is still connected to said first sheath, and further configured to cover said first sheath and said implant while said delivery system is retracted and withdrawn from said body. At least one release wire may extend from said proximal end of said first sheath to said distal end, wherein said release wire is configured to connect said implant to said first sheath. Said implant may be provided with at least one collapsible anchor, and further wherein said at least one release wire connects said implant to said first sheath by exiting at least one slot positioned along said first sheath, crossing over said anchor in said collapsed state, and entering the at least one slot or a second slot along said first sheath. Retraction of said at least one wire proximally may cause said implant to be released from said first sheath.

A reference sensor may be positioned along the first sheath or the second sheath, wherein the reference sensor is independent of said implant. Said reference sensor may be at least one of a: pressure sensor, a blood oxygen level sensor, a microphone, a sensor of tissue optical properties, a temperature sensor, a flow rate sensor, and a chemical sensor. Said system may be configured to allow a user to mechanically couple or decouple said first sheath and said second sheath, such that said sheaths can be made movable or non-movable with respect to one another during use. Said first sheath may be configured with a distal tip made of a soft material to minimize vessel trauma during use, wherein said distal tip has a durometer softer than Shore 40A.

**In** an example not covered by the claims, provided is a method for implanting an implant device in the vasculature of a human body, said method comprising the steps of: establishing access to the vasculature at an access location along the patient's anatomy; inserting a first catheter configured to translate from the access location to a target site within the vasculature; placing a guide wire between said access point to said target location; removing said first catheter while leaving said guide wire in place; inserting a delivery system over said guide wire, said delivery system comprising a first sheath and a second sheath each extending from a proximal end of said implant delivery system, wherein at least said first sheath extends to a distal end of said implant delivery system and wherein said first sheath is positioned at least partially within said second sheath, and includes an implant attached to said first sheath, wherein said first sheath or second sheath configured to allow the injection of contrast dye for angiographic imaging; advancing said delivery system to said target site while using angiographic imaging to position said implant at said target site; deploying said implant from said delivery system at said target site; and withdrawing said second catheter and said guidewire from said body while said medical device remains at said target location. Said delivery system may include a port configured to fluidically couple a portion of said first sheath or second sheath that is inside said vasculature to a portion of said first sheath or second sheath that is outside of said vasculature, said method further comprising the steps of: connecting said outside portion of said first sheath or second sheath to a pressure measurement device; measuring pressure at said inside portion of said first sheath or second sheath using said pressure measurement device; measuring pressure at said inside portion with said implant; comparing said measurement from said pressure measurement device to a measurement made by said implant. The method may further comprise the steps of assessing an accuracy of said implant and calibrating said implant.

Said port may be configured to inject contrast dye within the vasculature. Said implant may be connected to an exterior surface of said first sheath and positioned near a distal end of said second sheath, and wherein said first sheath and said second sheath are movable with respect to one another to deploy said implant to the target site. Said first catheter device comprises one of a balloon member configured to follow vascular flow to translate from said access location to said target site and a steerable tip catheter.

Also provided is a method for implanting an implant in a vasculature of a body, said method comprising the steps of: establishing access to the vasculature at an access location; inserting a catheter device into said vasculature at said access location, said catheter device fitted with said implant and configured to advance to a target site; advancing said catheter device to said target site; deploying said implant from said catheter device at said target site; and withdrawing said catheter device from said body while said implant remains at said target site. Said catheter device may be configured to advance to said target site by a balloon member configured to follow vascular flow to translate from said access location to said target site and a steerable tip catheter. Said catheter device may be configured to inject contrast dye into said vasculature to facilitate angiographic imaging, said method further comprising the step of advancing said catheter to said target location while using angiographic imaging. Said catheter device may be configured to fluidically couple a portion of said catheter device inside said vasculature to a portion of said catheter device that is outside of said vasculature, said method further comprising the steps of: connecting said of said catheter device that is outside of said vasculature to a pressure measurement device; before withdrawing said catheter device from said body, measuring pressure at said portion of said catheter device inside said vasculature using said pressure measurement device; measuring pressure using said implant; comparing said measurement from said pressure measurement device to a measurement made by said implant.

Said catheter device comprises a first sheath and a second sheath each extending from a proximal end of said catheter device, wherein at least said first sheath extends to a distal end of said implant delivery system, wherein said first sheath is positioned at least partially within said second sheath; wherein said implant is connected to an exterior surface of said first sheath and positioned near a distal end of said second sheath, and wherein said first sheath and said second sheath are movable with respect to one another to deploy said implant to a desired target location.

Also provided is an implant delivery system comprising: an implant; a first sheath and a second sheath each extending from a proximal end of said implant delivery system, wherein at least said first sheath extends to a distal end of said implant delivery system, wherein said first sheath is positioned at least partially within said second sheath; wherein said implant is connected to an exterior surface of said first sheath and positioned near a distal end of said second sheath; and wherein said first sheath and second sheath are moveable with respect to one another to deploy said implant to a desired location. Said implant further comprises a rigid housing, and at least one collapsible implant anchor, said at least one anchor being attached to said housing. Said anchor may be comprised of at least one wire that passes through at least one hole that extends through the thickness of said housing; and an enlarged portion of the wire is placed along said wire to retain the wire within the hole. Said hole may be counter-bored, said counter-bored hole comprising a first size hole and a second size hole, wherein the first size is smaller than the second size, wherein the second hole extends only partially through said housing, and further wherein said enlarged portion is configured to fit into said second hole. Each end of said anchors may be attached through a hole in said housing, such that said anchor forms a partial or complete loop including two ends that terminate at points on said housing. Said implant includes a marker configured to be at least partially visible under fluoroscopic imaging. Said marker may be positioned within said hole and includes a radio opaque material. Said anchor wire includes a nitinol material with a platinum core. Said marker may be a radio opaque marker that includes one of a: paint, ink, or preformed cylindrical tube. The marker may be a tube attached to said anchor wire by one of: adhesive, heat shrinking, and friction fit. A plurality of said markers may be spaced at known intervals along said anchor wire or said catheter device to facilitate distance estimation during angiographic imaging. Said marker may be located on the anchor wire near a point where said anchor wire attaches to said housing. Said hole may be filled with a filler material to prevent rotational or translational movement of said anchor relative to said hole, wherein said filler material is selected from: adhesive, potting material, epoxy, silicone, or polymer. A plurality of said markers comprise an asymmetric pattern on said implant body, said pattern configured to facilitate determination of implant orientation when viewed on a fluoroscope. Said asymmetric pattern comprises three marks positioned along three corners of a two-dimensional rectangle when viewed on a fluoroscope.

Further, provided is a method for attaching a wire anchor to a rigid implant body, comprising the steps of: providing at least one hole that extends through an implant housing; passing an anchor wire through said hole; enlarging at least one portion along said wire to prevent said enlarged portion from passing through said hole. Said hole may be formed by counter-boring to create a large portion and a small portion. Said enlarged portion of said wire may be placed into said large portion of said hole. At least one preformed fluoroscopic marker may be placed over said anchor wire prior to enlarging said dimension of said terminal end. Two ends of said wire anchor may be attached to said implant body such that said anchor forms a loop. Said hole may be filled with a filler material to event translational or rotational movement of said anchor.

### DESCRIPTION OF THE DRAWINGS

These, as well as other objects and advantages of this application, will be more completely understood and appreciated by referring to the following more detailed description of the presently preferred exemplary embodiments of the application in conjunction with the accompanying drawings, of which:
Figure 1 illustrates the schematic diagram of a prior art procedure for deploying a sensor to target site within a patient's anatomy;
Figure 2 illustrates an embodiment of an implant attached to a delivery system according to aspects of the instant application;
Figure 3 illustrates the schematic diagram of an embodiment of a method and system according to aspects of the instant application for deploying a sensor to target site within a patient's anatomy;
Figure 4 illustrates the schematic diagram of another embodiment of a method and system according to aspects of the instant application for deploying a sensor to a target site within a patient's anatomy;
Figure 5 illustrates the schematic diagram of another embodiment of a method and system according to aspects of the instant application for deploying a sensor to a target site within a patient's anatomy;
Figure 6A illustrates an embodiment of an implant from Figure 22 of commonly owned US Patent No 10,206,592;
Figure 6B is a cross sectional view of attachment portions of an implant according to aspects of the instant application;
Figure 7A is a cross sectional view of a similar implant to Figure 6B that includes an anchor wire as it is being assembled to the implant according to according to aspects of the instant application;
Figure 7B is a cross sectional view of an embodiment of the implant of Figure 7A that includes the anchor wire with a marker band as it is being assembled to the implant according to according to aspects of the instant application;
Figure 7C is a cross sectional view of an embodiment of the implant of Figure 7A that includes the anchor wire with the marker band as it is being assembled to the implant according to according to aspects of the instant application;
Figure 7D is a cross sectional view of an embodiment of the implant of Figure 7A with the anchor wire and marker band as it is being assembled to the implant according to according to aspects of the instant application;
Figure 7E is a cross sectional view of an embodiment of the implant of Figure 7A with the anchor wire and marker band assembled to the implant according to according to aspects of the instant application;
Figure 8A illustrates a schematic plan view of an embodiment of the implant that includes marker band locations according to aspects of the instant application;
Figure 8B illustrates a schematic plan view of an embodiment of the implant that includes marker band locations according to aspects of the instant application;
Figure 8C illustrates a schematic plan view of an embodi9ment of the implant that includes marker band locations according to aspects of the instant application;
Figure 9 is a cross sectional schematic view of an implant attached to a delivery system according to aspects of the instant application; and
Figure 10 illustrates an embodiment of an implant attached to a retrieval system according to aspects of the instant application.

### DETAILED DESCRIPTION

Reference will now be made in detail to exemplary embodiments of the present teachings, examples of which are illustrated in the accompanying drawings. It is to be understood that other embodiments may be utilized and structural and functional changes may be made without departing from the respective scope of the present teachings. Moreover, features of the various embodiments may be combined or altered without departing from the scope of the present teachings. As such, the following description is presented by way of illustration only and should not limit in any way the various alternatives and modifications that may be made to the illustrated embodiments and still be within the spirit and scope of the present teachings. In this disclosure, any identification of specific shapes, materials, techniques, arrangements, etc. are either related to a specific example presented or are merely a general description of such a shape, material, technique, arrangement, etc.

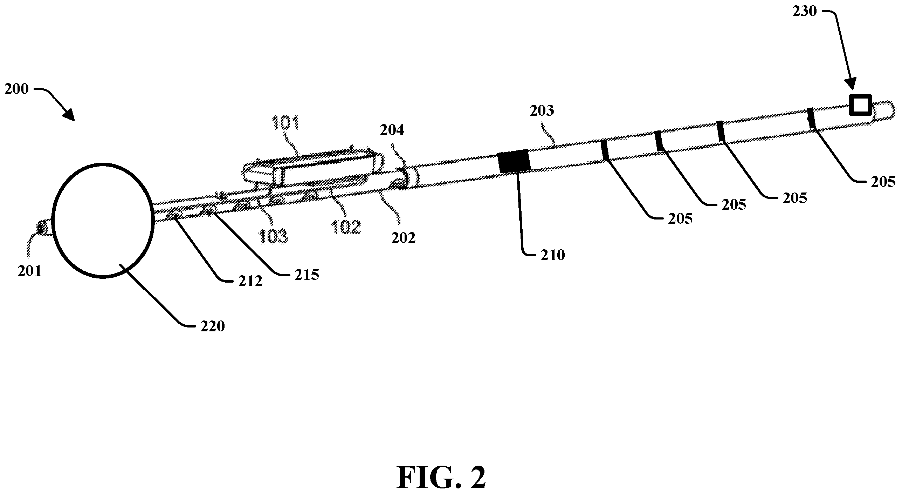

The instant application is directed to a method and system of utilizing a version of the delivery device similar to that disclosed by US Patent US Patent No. 10,206,592 of which this claims priority from. Referring now to the Figures, wherein common elements are identified by the same numbers, Figure 2 illustrates a perspective view of a portion of such an improved delivery device and system 200. The instant application discloses a catheter that includes a first or inner/carrier sheath 202 with a distal tip 201 and a second or outer/torque sheath 204 that extends proximally therefrom. The sheaths include one or more lumens that extend therein to perform a variety of functions including but not limited to: support a guide wire, allow for fluid passage, support release wires, and inflate or deflate a balloon positioned along the length of the delivery device. The first and second sheaths 202, 204 may be elongated and be attached at a proximal end to a handle (not shown) to allow for the control of rotation and translation relative to one another. The carrier sheath 202 and torque sheath 204 may be temporarily fixed to each other such that fixed rotation and translation occurs when desired, but when not desired, carrier sheath 202 and torque sheath 204 may move relative to one another.

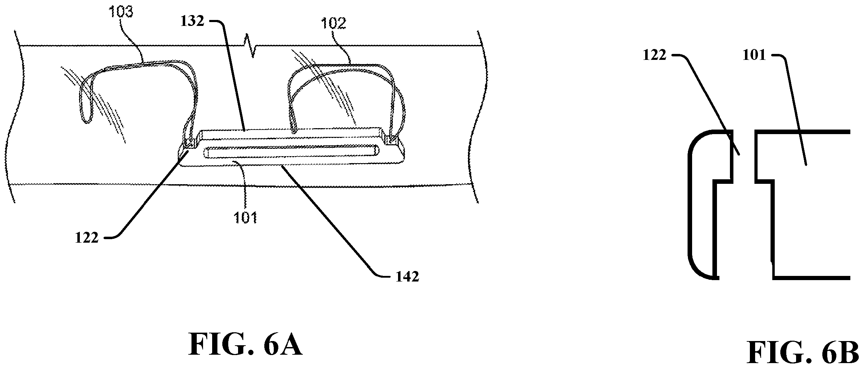

One or more anchor release wires 212 may extend within lumens within the sheaths 202, 204. The wires 212 may extend from a proximal end of the catheter towards the distal end and are configured to temporarily support and attach an implant 101 to the carrier sheath 202. This attachment may be established by engagement of anchors that extend from the implant 101. In one embodiment, the implant may be a wireless device configured to electronically communicate with a receiver or external reader device. The implant may include an LC resonant tank within a housing. In another embodiment, the implant may be a passive device or an active device and include a battery or other power source. The anchors may include a distal anchor 103 and proximal anchor 102 that extend from opposing portions of the implant 101. The anchor release wires 212 attach to the carrier sheath 202 and anchor 102, 103 by entering into and out of one or more slots 215 positioned along the carrier sheath 202. This arrangement may allow the anchors to be collapsed during implantation and to expand (e.g., Figure 6A) once the release wires 212 have been removed.

The carrier sheath 202 may be configured to rotate and to translate with the torque sheath 204 or may be able to rotate and translate relative to the torque sheath 204. This allows the sheaths to move proximal/distal relative to one another or to prevent rotation of one sheath relative to the other. Further, the distal tip 201 as well as desired portions along the sheaths 202, 204 may be covered with radio opaque material to improve visibility during fluoroscopy. Likewise a ruled set of radio opaque markers 205 may be placed along a portion of the sheaths 202, 204 having pre-defined separation distances. These markers 205 may assist with establishing visible cues to assist the medical personnel with placement of the delivery system 200 within the anatomy.

The outer sheath 204 may carry out various functions that are different from prior versions of known systems. These functions include (a) injection of contrast dye for real-time angiography at any time when the outer sheath 204 is in the body; (b) use the outer sheath as the fluid column (aka 'fluid channel' aka 'lumen') that fluidically couples the distal end of the outer sheath to a proximal port 230, positioned outside the patient's body, (c) a pressure transducer (not shown) may be fluidically attached to the proximal port for procuring reference measurements, and (d) a reference sensor 210 may be attached to a distal portion of the outer sheath 204 for procuring reference measurements. Additionally, a balloon member 220 may optionally be positioned along the length of the inner sheath 202 or outer sheath 204 which may be toggled to inflate or deflate to assist with guiding the delivery device within the vasculature as described below. Said balloon member may be configured to be inflated to guide said deliver system to the target site. Further, said balloon member may be configured to facilitate a wedge pressure measurement. Said balloon member may also be configured to limit blood flow in a vessel to facilitate implant deployment retraction, or rotation while it may also be configured to hold said first sheath in place with respect to a blood vessel while said implant is released from said first sheath at said target site. Said fluid port may be configured to allow removal of fluid from said body. At least one temperature sensor may be positioned on said distal end/ tip 201 to facilitate measurement of flow rate by thermodilution, wherein said flow rate measurement is configured to determine a cardiac output. Said second sheath may be configured to allow insertion of a catheter device, wherein said catheter device includes at least one of: a camera, a pressure sensing catheter, a stent placement device, a valve placement device, a microphone, an ablation device, a balloon device, a Swan Ganz catheter, an electrical stimulation device, an ultrasound device, a drug delivery device, a catheter for gripping implanted devices, a catheter for readjusting the position of implanted devices, a catheter for removing implanted devices.

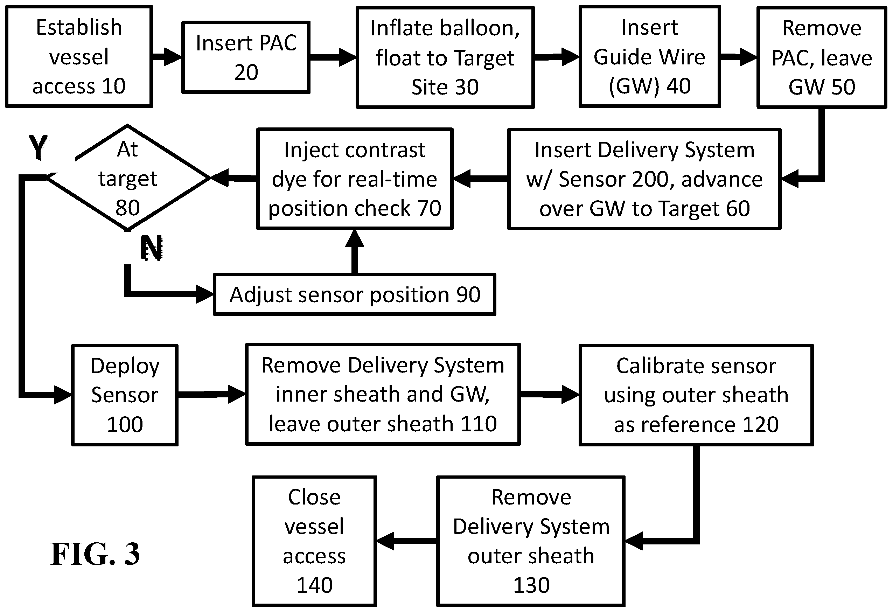

Figure 3 illustrates a schematic diagram of an embodiment of a method and system according to aspects of the instant application for deploying a sensor or implant to a target site within a patient's anatomy. This disclosure contemplates the use of the delivery system 200 for surgically implanting the implant 101 or sensor within the vascularity of a patient to communicate with a reader device (not shown). Various embodiments of the implant/sensor and reader device are contemplated by the following commonly owned patent documents: U.S. Patent App. No. 15/958,613 entitled "ANCHORING SYSTEM FOR A CATHETER DELIVERED DEVICE," filed on April 20, 2018; U.S. Patent App. No. 15/213,712 entitled "PRESSURE SENSING IMPLANT," filed on July 19, 2016; U.S. Patent Application No. 14/777,654 entitled "PRESSURE SENSING IMPLANT" filed on September 16, 2015; U.S. Patent No. 8,493,187 entitled "WIRELESS SENSOR READER" filed on March 19, 2010; U.S. Patent No. 8,899,582 entitled "CARDIAC PRESSURE MONITORING DEVICE" filed on January 25, 2008; PCT Patent App. No. PCT/US2012/044998 entitled "IMPLANTABLE SENSOR ENCLOSURE WITH THIN SIDEWALLS" filed on June 29, 2012, PCT Patent App. No. PCT/US2011/045581 entitled TRANSVASCULAR WIRELESS SENSOR SYSTEM" filed on July 27, 2011; U.S. Patent App. No. 15/958,613 entitled "ANCHORING SYSTEM FOR A CATHETER DELIVERED DEVICE," filed on April 20, 2018; U.S. Provisional Application No. 62/624,146 entitled "DEVICE AND METHOD FOR DEPLOYING AND SECURING AN IMPLANT TO A VESSEL WALL," filed on January 31, 2018. However, this application is not limited to the use with these devices or assemblies as others may be contemplated.

Steps 10, 20 and 30 are comparable to steps (1), (2) and (3) of Figure 1. Venous access may be established at an access location along the patient's anatomy by puncturing a vein percutaneously 10. This may also include inserting an "introducer" at the access location to support the insertion of various catheters. The femoral vein is a typical access location; jugular, brachial, and subclavian vein are also known access locations. The distal end of a first pulmonary artery catheter ("PAC") is then inserted at the access location 20. A balloon may be inflated along a distal portion or distal top of the PAC within the vascularity of the patient 30.

Notably, step (4) of Figure 1 is not necessary for the process of Figure 3, this is because a contrast map for future use is not appropriate as the anatomy is to be mapped in real time when the implant 101 is near the target site. Here, the PAC includes thee balloon function, so any balloon catheter that can get you to the target site is acceptable - it does not need the additional contrast dye function.

Steps 40, 50, and 60, are similar to steps (5), (6), and (7) of Figure 1. Here, the distal end of the PAC may be located at or near the target site and a guide wire (GW) may be inserted or positioned within a lumen of the PAC 40. The guide wire (GW) may slide or otherwise be withdrawn from a lumen within the PAC to be positioned at or adjacent the target site 50. The PAC may be removed allowing the guide wire (GW) to remain positioned therein. The guide wire (GW) may remain located within the vasculature between the access location and the target site to guide other catheters in and out to the target site 60. Notably different from step (7) is that the instant delivery system with sensor 200 are advanced over the guide wire (GW) to the target site.

The delivery system 200 allows for contrast dye to be injected therefrom to allow for a real-time imaging (fluoroscopy imaging) of the location of the delivery system 200 and implant 101 or sensor relative to the anatomy of the patient 70. This allows for the injection of contrast dye to be performed while the implant 101 is actually near the target site. Further, the radio-opaque markings 205 provide imaging cues to allow the medical practitioner to view and confirm exactly where the delivery system 200 and implant 101 are positioned and adjusted relative to the actual target site or other benchmark locations within the anatomy with live fluoroscopic imaging (steps 80 and 90). This method does not require the medical practitioner to compare the relative position of a catheter viewed in the fluoroscopic image with a static angiogram image acquired when contrast dye was introduced by the PAC moments ago. This step reduces the guess work or estimating of sizes and locations using the static image of the angiogram in the estimation of placing the sensor or implant at the target site in the anatomy.

These steps do not require the use of an anatomic reference marker like ribs, spine, or other implants to estimate where the implant 101 may be positioned or adjusted relative to the target site. This process improves accuracy and reduces the risk of releasing the implant or sensor in an incorrect location (which could be a safety problem). This method may also benefit when the distal anchor and/or proximal anchor include shapes as disclosed by U.S. Patent App. No. 15/958,613 wherein at least one anchor includes an elongated and angled orientation relative to the implant 101 or the at least one anchor may include a clover-shaped structure. This real-time angiography feature, and the configuration of the anchor's relative to the implant 101 may allow a medical practitioner to confidently move and adjust the implant 101 to the best available location in the anatomy prior to its placement. Once the implant is positioned at the target site 80 and adjusted in the best location 90, the implant 101 or sensor may be deployed 100. The implant 101 may be deployed by the medical professional by pulling the release wires 212 thereby retracting the release wires 212 from the distal anchor and the proximal anchor to allow the anchors to expand and anchor the implant in place within the target site.

The inner sheath 202 may be removed from or partially retracted relative to the outer sheath 204 and the anatomy of the body 110. Optionally, the guide wire (GW) may also be removed, it may remain in place, or may be partially removed to provide some stability for the outer sheath 204. A calibration of the implant 101 may be performed 120. Here, a port 230 positioned along a proximal portion (outside the body) is connected to a pressure transducer, so that is it fluidically coupled to the bloodstream, at the distal portion of the second sheath 204. The pressure in the blood vessel is communicated to the port 230 and pressure transducer to provide an accurate reference pressure measurement. Notably, a second catheter is not needed (as is required in the Figure 1 method). The calibration may be performed by comparing a pressure measurement reading taken by the pressure transducer with a pressure measurement reading taken by the implant 101 deployed at the target site. The pressure measurement reading taken by the implant 101 may be wirelessly communicated to a receiver or reader device. One example of a reader device is disclosed by U.S. Patent No. 8,493,187 and its related patents. The deployed implant 101 may be adjusted by mathematically changing calibration coefficients or by using a lookup table and programming or adjusting the reader device or implant 101. The outer sheath 204 may then be removed from the anatomy 130 and the access location may be closed 140.

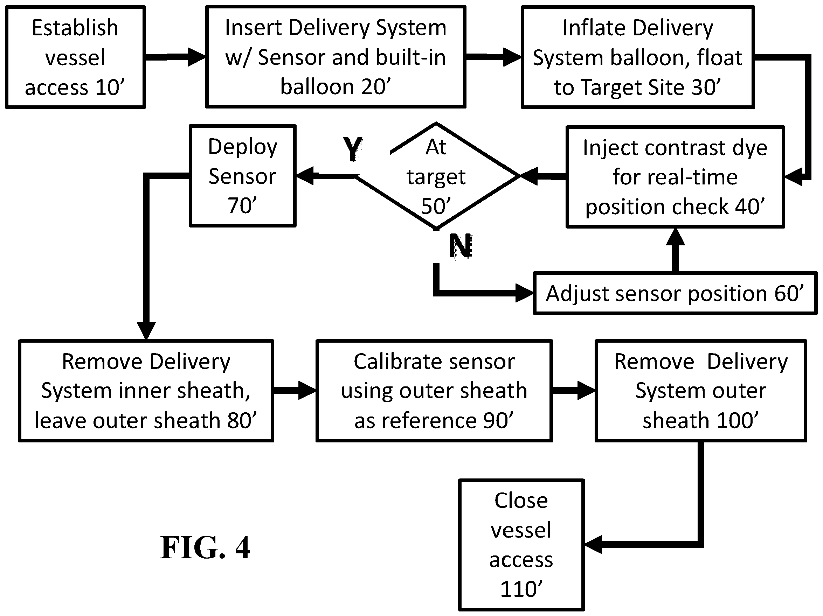

Figure 4 illustrates another embodiment and assumes the use of a different type of delivery system catheter than the type described in Figures 1 or 3. This catheter includes a two-sheath concept as in Figure 2, but also includes the balloon member 220 positioned along a distal portion of the inner sheath 202. This method reduces the need for utilizing a PAC to position the guide wire at the target site of steps (2) and (3) or steps 20 and 30 or to otherwise carry out the procedure.

Step 10' is comparable to step (1) of Figure 1 and step 10 of Figure 3. However, after venous access is established at the access location along the patient's anatomy (10'). The distal end of a delivery system 200 with balloon member 220 and implant 101 is then inserted at the access location (20'). The balloon may be inflated along a distal portion or distal top of the delivery system 200, preferably along the inner sheath 202 within the vascularity of the patient (30'). In this embodiment, there is no need for a guide wire (GW).

Steps 40', 50', and 60', are similar to steps 70, 80, and 80 of Figure 3. The delivery system 200 allows for contrast dye to be injected therefrom to allow for a real-time imaging (fluoroscopy imaging) of the location of the delivery system 200 and implant 101 or sensor relative to the anatomy of the patient 40'. This allows for the injection of contrast dye to be performed while the implant 101 is actually near the target site. Further, the radio-opaque markings 205 provide imaging cues to allow the medical practitioner to view and confirm exactly where the delivery system 200 and implant 101 are positioned and adjusted relative to the actual target site or other benchmark locations within the anatomy with live fluoroscopic imaging (steps 50' and 60'). The remaining steps 70' - 110' are comparable to steps 100-140 of Figure 3.

Once the implant is positioned at the target site 50' and adjusted in the best location 60', the implant 101 or sensor may be deployed 70'. The implant 101 may be deployed by the medical professional by pulling the release wires 212 thereby retracting the release wires 212 from the distal anchor and the proximal anchor to allow the anchors to expand and anchor the implant in place within the target site.

The inner sheath 202 may be removed from or partially retracted relative to the outer sheath 204 and the anatomy of the body 80'. A calibration of the implant 101 may be performed 90'. The outer sheath 204 may then be removed from the anatomy 100' and the access location may be closed 110'.

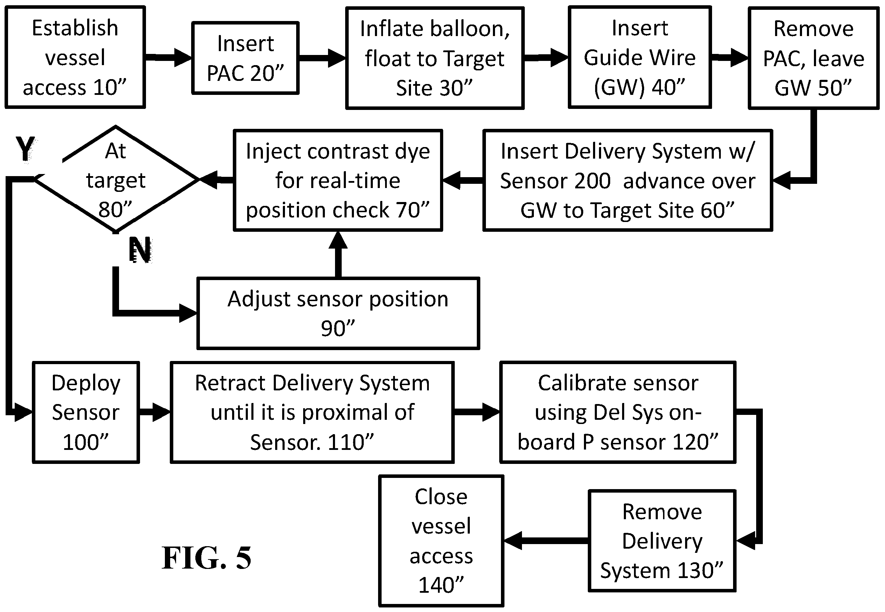

Figure 5 illustrates another embodiment and assumes the use of a different type of delivery system catheter than the type described in Figures 1, 3, and 4. Here, this delivery system 200 includes a two-sheath concept as in Figure 2, but instead of the pressure transducer and the balloon member 220, a reference sensor 210 may be positioned along a distal portion of the outer sheath 204. Steps 10"-140" are otherwise comparable to steps 10-140 of Figure 3. Figure 2 illustrates use of the reference sensor 210 (separate from the implant 101) that is attached along a distal portion of the outer sheath 204. The reference sensor 210 may be powered by a battery and operate wirelessly to communicate pressure data to an outside receiver. Optionally, the reference sensor 210 may be connected to circuitry at the proximal end of the delivery system 200 by contact wires. In this embodiment, the reference sensor 210 is utilized in lieu of the pressure transducer and associated fluid port 230 of the outer sheath 204 (of Figure 3) is not needed to perform the calibration step. Calibration step 120" may thus be performed by the reference sensor 210 that is configured to wirelessly communicate information to a receiver or reader device. This disclosure also contemplates an embodiment of the delivery system 200 that includes the balloon member 220 as well as the reference sensor 210 in which the featured steps of the self-placement of the delivery system 200 with balloon member 200 of Figure 4 may be combined with the reference sensor 210 based calibration of Figure 5.

Further, Figure 6A illustrates an implant 101 from Figure 22 of commonly owned US Patent No 10,206,592 in which distal and proximal anchors 103, 102 are positioned in an expanded configuration within the vasculature of a patient's anatomy. The anchors are disclosed to extend from opposing ends of the implant 101 and are attached to the implant 101 at attachment portions 122. Figure 6A illustrates the implant 101 includes an inner facing surface 132 configured to face inwardly of the vasculature and an opposite outer facing surface 142 configured to abut against the inner wall of the anatomy. Figure 6B is a cross sectional view of the implant 101 according to embodiments of the instant application that illustrates a novel way to configure the attachment portions 122.

The markers 205, discussed above, may be radio opaque to assist medical practitioners to identify a location of a device during fluoroscopy (i.e., x-ray imaging). The markers may be made from radio-opaque chemical compounds which can be added to polymers, ceramics, plastics, or other materials for molding into various shapes. There also exists radio-opaque coatings or inks that can be applied to surfaces. A common product for use in catheters is radio-opaque tubing, such as supplied by Zeus, Polyzen, or Fluortek Corporation.

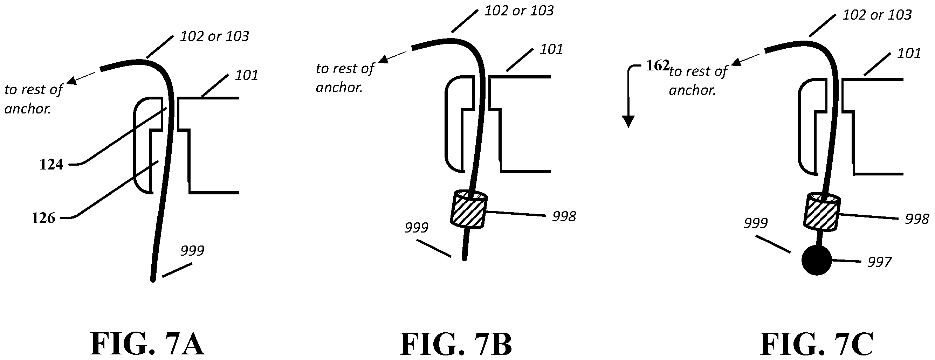

Figures 7A - 7E illustrate an embodiment of the instant disclosure that identifies an improvement over known embodiments of implants that are configured to include markers thereon. In an embodiment of the disclosure, the markers may include a cylindrical shape length of a marker or tube 998 having radio-opaque material thereon. The marker 998 may be a tube cut into a cylinder shape of desired length and attached to a portion of the anchor by a heatshrink, adhesive, or other process such as friction fit.

This disclosure illustrates an improved way to attach and configure markers to assist in fluoroscopic imaging to an implant 101. In an embodiment, a plurality of through holes may be drilled through portions of the implant 101. There may be any number of through holes but in the disclosed embodiment, four holes are provided to include four attachment points 122 to the implant for use with the distal and proximal anchors 103, 102. The through holes may be counter-bored having two overlapping holes of different diameters. A first hole 124 being a through hole having a first size, and a second hole 126 only extending partially through the implant body and having a second size. The first hole 124 may be concentric relative to the second hole 126. **In** this embodiment, the first size may be smaller than the second size thereby creating a shoulder or stop between the first hole 124 and second hole 126. **In** Figure 7A, a terminal end 999 of anchor wire 102 or 103 may be threaded through the first and second holes 124, 126, with the terminal end exiting through the large sized second hole 126. Figure 7B illustrates the marker 998 as a tube attached along the anchor wire adjacent to a terminal end of the anchor. The marker 998 may be a cylindrical shaped radio opaque marker tube 998 that is slid over the free end of the anchor. The marker 998 may sit freely along the wire or may be adhered, heat shrunk, crimping, or friction fit thereon. Note that the marker may be any shape and is not limited to being cylindrical.

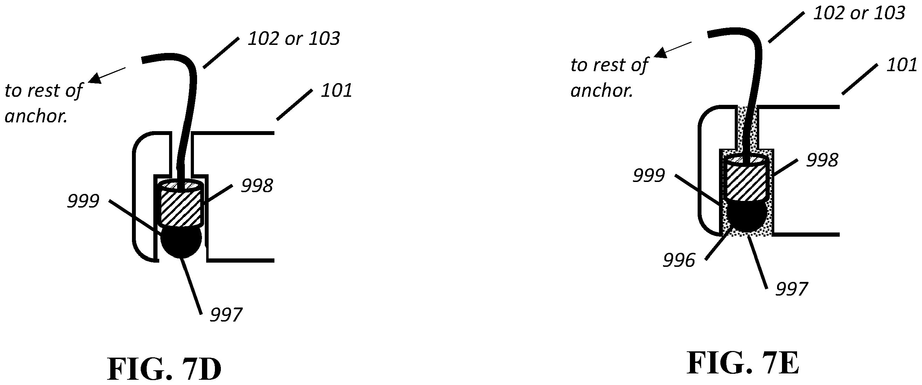

Figure 7C illustrates an enlarged end 997 positioned on the terminal end 999 of the wire of the anchor 102/103. The enlarged end 997 and marker 998 are configured to be positioned within the larger sized second hole 126 while the remaining portion of the anchor extend through the smaller sized first hole124. As such, at least the enlarged end 997 may abut against the shoulder and may not exit through the small sized first hole 124. The enlarged end 997 may be formed by: heating the end to form a ball; using a laser; stamping; "coining" or otherwise compressing the end to flatten it; bending the end to form a coil shape, or otherwise enlarging it by means known to those skilled in the art. Figure 7D illustrates the assembly having larger materials fit snugly into the large size second hole 126. The enlarged end 997 ensures that the wire cannot exit the first hole 124, increasing system safety and reliability. In Figure 7E, the remaining space in the holes 124, 126 may be filled with a filler material 996, typically adhesive, potting material, epoxy or other filler material that may be safe for human implantation. As implant 101 may be made of a glass or ceramic material, the concentric through hole configuration is not known to be used in an implant assembly, method of manufacture, or delivery system. The embodiments of the marker and implant 101 assembly may include various alternate designs including: (i) without a marker band 998, but use of a radio opaque filler material 996; (ii) no marker band; (iii) crimp-on marker band 998 and no enlarged end (the crimp-on holds the wire in place); (iv) no enlarged end, filler material holds the assembly in place. Notably, two ends of said wire anchor may be attached to said implant body such that said anchor forms a loop and the counter bored hole may be filled with a filler material to prevent translational or rotational movement of said anchor.

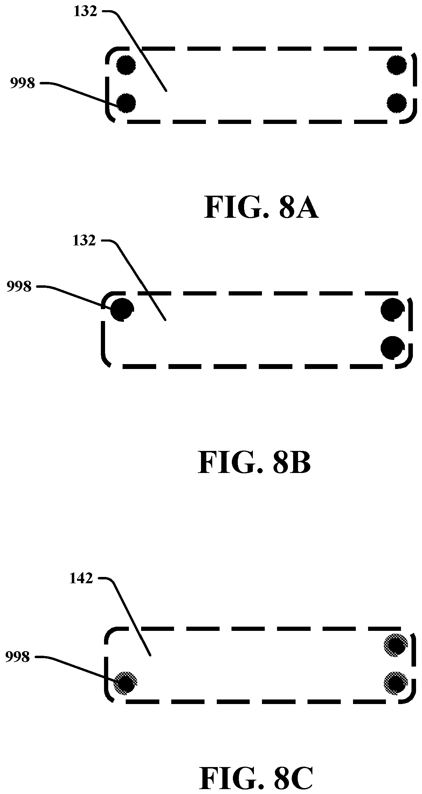

Figures 8A, 8B, and 8C illustrate the concept of providing symmetrical or asymmetrical radio opaque markings. As can be seen in Figure 6A, the implant body, shown in side view, includes an inner facing surface 132 configured to face inwardly of the vasculature and an opposite outer facing surface 142 configured to abut against the inner wall of the anatomy. The inner facing surface 132 may include a sensitive diaphragm desirable to keep away from a vessel wall and facing towards the bloodstream to be able to accurately sense or measure pressure. Figure 8A illustrates an example for better understanding the invention, wherein markers 998 are placed in four corners to allow a fluoroscope image to view the implant 101 to include four dots. The hashed outline of the implant body is for reference only and would likely not be visible on the fluoroscope image. According to the invention, the plurality of said markers comprise an asymmetric pattern on said implant body, said pattern configured to facilitate determination of implant orientation when viewed on a fluoroscope. Said asymmetric pattern includes three marks positioned along three corners of a two-dimensional rectangle when viewed on a fluoroscope.

By making the dot pattern asymmetrical, as in Figure 8B, the fluoroscope image can identify which surface (inner facing surface 132 or outer facing surface 142) and relative position of the implant 101 may be viewable. The asymmetrical pattern includes placing markers 998 in three of the four corners. In this way, the medical practitioner may easily identify which surface is facing an imaging device to allow for precise adjustment and positioning the implant at the target site in the anatomy.

Figure 2 illustrates various locations to place markers 205 on either or both the implant 101 and the sheaths 202, 204 of the delivery system 200. These locations include but are not limited to the distal tip 201; distal end of the second sheath 202; a set of ruled stripes along first sheath 204 or second sheath 202. The ruled stripes could be evenly spaced or unevenly spaced. These marker arrangements can help serve as a ruler to judge distances, positions, and adjustments during placement of the implant by viewing live images on the fluoroscope.

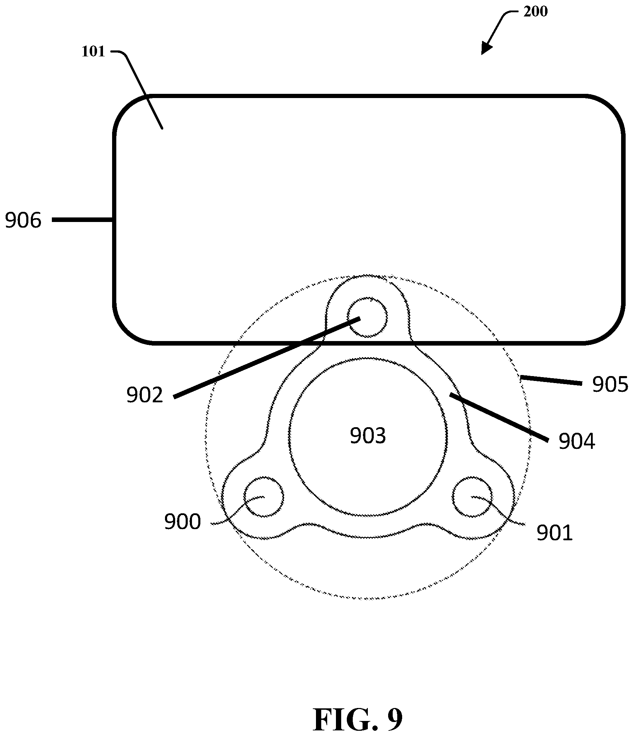

Figure 9 illustrates an embodiment of the delivery system 200 in cross section having the implant 101 mounted thereon as viewed towards the distal end. The inner sheath 202 may include a plurality of lumens aligned along its length. In one embodiment, the inner sheath 202 includes general triangle shape cross section with a plurality of outer lumens 900, 901, and 902 spaced from one another at about 0°, 120°, and 240° and extruded along its length. A center lumen 903 may be positioned along centrally from the outer lumens. Any number of lumens, including one, two, three, four, five, six, seven, or more are contemplated by this disclosure. Each of the plurality of lumens may extend the entire length of inner sheath 202. Additionally, one or more of the lumens may include slots or flattened portions to assist with attachment of the implant 101 thereto.

The hashed line represents an outer perimeter of the outer sheath 204. Part of the inner sheath 202 may be contained inside the outer sheath 204 (not shown except as hashed line), and part of the inner sheath 202 may extend outside the outer sheath 204 as illustrated by Figure 2. The large rectangle 906 represents a body of the implant 101. The body 906 may rest on a flattened region (as a portion of lumen 902 may be cut or flattened out) on the portion of inner sheath 202 that extends outside of outer sheath 204. The center lumen 903 may be larger than the outer lumens 900, 901, 902 and may be configured to support the guide wire (GW) as described above.

Lumens 900 and 901, positioned along the underside of the inner sheath 202 may be to support the release wires 212. In one embodiment, the anchors 102, 103 may be folded down in a collapsed configuration and positioned partially around or tucked under the bottom of the inner sheath 202. The release wires 212 may be made of nitinol, stainless steel, or another extrudable material, and may be contained in lumens 900 and 901. The release wires 212 may be threaded through various slots 215 as identified in Figure 2 to hold the anchors 102, 103 in the collapsed configuration. In an embodiment, the release wires 212 may be pulled proximally from the proximal end of the delivery system (where there may be a handle). As ends of the release wires 212 move past each collapsed anchor 102, 103, the anchor is released and deploys to its unfolded or expanded shape. The anchors may each deploy one at a time or simultaneously.

Alternatively, the delivery system 200 may include a single continuous release wire 212 that aligns through a first lumen (e.g., 900), ties down anchors 102, 103 along a first side of the implant 101 and loops around the distal portion to tie down opposing sides of the anchors 102, 103 on the other side through a second lumen (e.g., 901). Here, the medical practitioner would pull just one end of that release wire 212 to release the anchors 102, 103 from the delivery system 200.

In one embodiment, lumen 902 is not used. In another embodiment, lumen 902 could be used for a third release wire that could support along a top side of implant body 906 to provide more stability and tie-down security. In another embodiment, lumen 902 could be used as a fluid column to connect to the pressure transducer and fluid port 230 located outside the body. Notably, the pressure transducer and fluid port 230 may be positioned along the inner sheath 202 or the outer sheath 204 but by using the inner sheath 202, a medical practitioner may be able to measure a pressure reading during the time the inner sheath 202 is within the body. In another embodiment, lumen 902 may be used to inject fluids such as contrast dye or drugs. Alternatively, the outer sheath 204 can be used to inject fluids such as contrast dye or drugs.

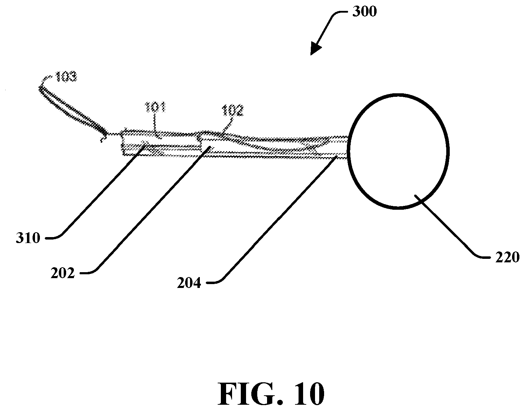

In another embodiment as illustrated by Figure 10, provided is an implant retrieval system 300 for removing an implanted device 100 from a human body. The implanted device having at least one portion (i.e., anchors 102, 103) that may be collapsible and wherein the first sheath 202 and second sheath 204 each extending from a proximal end and at least said first sheath extends to a distal end of the retrieval system 300. The first sheath 202 is positioned at least partially within said second sheath 204 wherein said first sheath 202 includes a linkage member 310 is configured to mechanically couple to a portion of said implanted device 101. The first sheath 202 and said second sheath 204 may be rotatably and translatable with respect to one another and said collapsible anchors 102, 103 of said implanted may be configured to return to a collapsed state when said first sheath 202 or said linkage member 310 retracts said implant 101 proximally into said second sheath 204.

The second sheath 204 of the retrieval system 300 may be configured to hold said collapsible portion 102, 103 of said implant 101 in said collapsed state while said system and said implant are withdrawn from said body. The retraction of said implant 101 may be accomplished by applying distally directed force to said second sheath 204 while applying a proximally directed force to said first sheath 202 or said linkage member 310 while said first sheath 202 is mechanically coupled to said implanted device 101. The retrieval system 300 may also include a balloon member 220 wherein said balloon member 220 may be configured to facilitate reducing blood flow during mechanical capture and retraction of said implanted device. The balloon member 220 may be configured to follow blood flow to initially guide said system to the location of said implanted device prior to retrieval.

The second sheath 204 (of either the retrieval system 300 or the delivery system 200) may be further configured to remain in place to allow exchanging the first sheath 202 for other catheter devices wherein said catheter device includes at least one of: a camera, a pressure sensing catheter, a stent placement device, a valve placement device, a microphone, an ablation device, a balloon device, a Swan Ganz catheter, an electrical stimulation device, an ultrasound device, a drug delivery device, a catheter for gripping implanted devices (linkage member 310), a catheter for readjusting the position of implanted devices, a catheter for removing implanted devices. The second sheath may also be configured to contain a plurality of sheaths in addition to said first sheath. The system 300 may also be configured to detach said implanted device 101 from surrounding tissue while said first sheath is mechanically coupled to said device wherein said detachment of said device from said surrounding tissue comprises cutting off a portion of said device and leaving said portion attached to said tissue as said device is retracted. Alternatively, the detachment of the implanted device from said surrounding tissue may include cutting off a portion of said surrounding tissue and leaving said portion attached to said device as said device is retracted. The detachment may be accomplished by one of: mechanical cutting, tissue ablation, local application of heat, local application of laser energy, local application of radio frequency (RF) energy, local application of ultrasonic energy, local application of vibrational energy.

Having described preferred embodiments of new and improved delivery system and implant configurations and methods, it is believed that other modifications, variations and changes will be suggested to those skilled in the art in view of the teachings set forth herein. It is therefore to be understood that all such variations, modifications and changes are believed to fall within the scope of the present application.

Although the embodiments of the present teachings have been illustrated in the accompanying drawings and described in the foregoing detailed description, it is to be understood that the present teachings are not to be limited to just the embodiments disclosed, but that the present teachings described herein are capable of numerous rearrangements, modifications and substitutions without departing from the scope of the claims hereafter. The claims as follows are intended to include all modifications and alterations insofar as they come within the scope of the claims.

## Claims

1. An implant delivery system (200) comprising:
an implant (101) including an inner facing surface (132) and an opposite outer facing surface (142);
a first sheath (202) and a second sheath (204) each extending from a proximal end of said implant delivery system (200), wherein at least said first sheath (202) extends to a distal end of said implant delivery system (200), wherein said first sheath (202) is positioned at least partially within said second sheath (204), the first sheath (202) is translatable relative to said second sheath (204);
wherein said implant (101) is connected to an exterior surface of said first sheath (202), and
wherein said first sheath (202) and said second sheath (204) are movable with respect to one another to deploy said implant (101) to a target site in an anatomy;
**characterised by**:
said implant (101) further comprising a plurality of markers (205, 998) configured to be visible with a fluoroscope, wherein the plurality of markers (205, 998) is positioned along said implant (101) in an asymmetric pattern, said pattern is configured to facilitate determination of implant orientation when viewed on a fluoroscope to determine which of the inner facing surface (132) or the opposite outer facing surface (142) may be viewable, and said asymmetric pattern comprises the markers (205, 998) at three of four corners of a two-dimensional rectangle when viewed normal to the plane of said rectangle on a fluoroscope.

2. The implant delivery system of claim 1, wherein said delivery system is configured to be partially inserted into a blood vessel of a human body such that said proximal end remains external to said body and said distal end is internal to said body.

3. The implant delivery system of claim 1, further comprising at least one fluid port (230) positioned along a proximal end of said first sheath (202) or second sheath (204), said fluid port (230) fluidly coupled to at least one lumen extending down the length of said first sheath (202) or second sheath (204) to allow fluid flow through said lumen.

4. The implant delivery system of claim 3, wherein said fluid is injected through said port and includes one of: a drug; a fluid used to enhance anatomical imaging; fluoroscopic contrast dye; barium; a radioactive material; blood; plasma; saline solution; a blood component; a particle suspension; a nano-device; and a nanomaterial.

5. The implant delivery system of claim 3, wherein said at least one fluid port (230) is further configured to operatively couple to a device located outside of said body.

6. The implant delivery system of claim 5, wherein said device is a pressure transducer, configured to measure a fluid pressure at the distal end of said first or said second sheath (204).

7. The implant delivery system of claim 6, wherein said measurement of fluid pressure at the distal end of said first or second sheath (204) is used to calibrate or assess the accuracy of said implant (101).

8. The implant delivery system of claim 1, wherein said markers (205, 998) include a radio opaque material positioned on at least one of: a distal tip (201) of said first sheath (202); the distal portion of said second sheath (204); a portion of said implant (101); and as a plurality of markings spaced along a portion of said first or said second sheath (202, 204).

9. The implant delivery system of claim 1, wherein said markers (205, 998) are attached to at least one anchor (102, 103) on the implant (101).

10. The implant delivery system of claim 1, wherein:
said second sheath (204) is configured to allow insertion of a catheter device configured to selectively attach to said implant (101) and move the implant (101) proximally when retracted;
wherein at least one anchor (102, 103) of said implant (101) is collapsible; and
wherein said anchor (102, 103) of said implant (101) is configured to be placed in a collapsed state when said catheter device moves the implant (101) proximally into said second sheath (204).

11. The implant delivery system of claim 1, wherein said second sheath (204) is further configured to allow said first sheath (202) to be retracted into said second sheath (204) while said implant (101) is still connected to said first sheath (202), and further configured to cover said first sheath (202) and said implant (101) while said delivery system is retracted and withdrawn from said body.

12. The implant delivery system of claim 1, wherein said implant (101) further comprises a rigid housing, and at least one collapsible implant anchor (102, 103), said at least one anchor (102, 103) being attached to said housing.

13. The implant delivery system of claim 12, wherein said anchor (102, 103) is comprised of at least one wire that passes through at least one hole (124, 126) that extends through the thickness of said housing; and an enlarged portion of the wire is placed along said wire to retain the wire within the hole (124, 126); and
wherein said hole (124, 126) is counter-bored, said counter-bored hole comprising a first size hole (124) and a second size hole (126), wherein the first size is smaller than the second size, wherein the second hole (126) extends only partially through said housing, and further wherein said enlarged portion is configured to fit into said second hole (126).

14. The implant delivery system of claim 13, wherein a plurality of said markers are spaced at known intervals along said anchor wire to facilitate distance estimation during angiographic imaging; and wherein said marker is located on the anchor wire near a point where said anchor wire attaches to said housing.

## Patentansprüche

1. Ein Implantat-Abgabesystem (200), das Folgendes umfasst:
ein Implantat (101), das eine nach innen gewandte Fläche (132) und eine gegenüberliegende nach außen gewandte Fläche (142) beinhaltet;
eine erste Ummantelung bzw. Hülle (*sheath*) (202) und eine zweite Hülle (204), die jeweils von einem proximalen Ende des Implantat-Abgabesystems (200) ausgehen, wobei sich zumindest die genannte erste Hülle (202) zu einem distalen Ende des genannten Implantat-Abgabesystems (200) erstreckt, wobei die genannte erste Hülle (202) zumindest teilweise innerhalb der genannten zweiten Hülle (204) positioniert ist, wobei die erste Hülle (202) relativ zur genannten zweiten Hülle (204) verschiebbar ist;
wobei das genannte Implantat (101) mit einer Außenfläche der genannten ersten Hülle (202) verbunden ist, und
wobei die genannte erste Hülle (202) und die genannte zweite Hülle (204) relativ zueinander beweglich sind, um das genannte Implantat (101) an einer Zielstelle in einer Anatomie einzusetzen;
**dadurch gekennzeichnet, dass**
das genannte Implantat (101) ferner Folgendes umfasst: eine Vielzahl von Markern (205, 998), die konfiguriert sind, um mit einem Fluoroskop sichtbar zu sein, wobei die Vielzahl von Markern (205, 998) entlang des genannten Implantats (101) in einem asymmetrischen Muster positioniert ist, wobei das genannte Muster dazu konfiguriert ist, die Bestimmung der Ausrichtung des Implantats zu erleichtern, wenn dieses auf einem Fluoroskop betrachtet wird, um festzustellen, welche von der nach innen gewandten Fläche (132) oder der gegenüberliegenden nach außen gewandten Fläche (142) sichtbar sein könnte, und wobei das genannte asymmetrische Muster die Marker (205, 998) an drei von vier Ecken eines zweidimensionalen Rechtecks umfasst, wenn durch ein Fluoroskop normal zur Ebene des genannten Rechtecks geschaut wird.

2. Das Implantat-Abgabesystem nach Anspruch 1, wobei das genannte Abgabesystem dazu konfiguriert ist, teilweise in ein Blutgefäß eines menschlichen Körpers eingeführt zu werden, sodass das genannte proximale Ende außerhalb des genannten Körpers verbleibt und das genannte distale Ende sich innerhalb des genannten Körpers befindet.

3. Das Implantat-Abgabesystem nach Anspruch 1, das ferner mindestens einen Fluid-Port (230) umfasst, der entlang eines proximalen Endes der genannten ersten Hülle (202) oder der genannten zweiten Hülle (204) positioniert ist, wobei der genannte Fluid-Port (230) mit mindestens einem Lumen fluidgekoppelt ist, das sich über die Länge der genannten ersten Hülle (202) oder der genannten zweiten Hülle (204) erstreckt, um einen Fluidfluss durch das genannte Lumen zu ermöglichen.

4. Das Implantat-Abgabesystem nach Anspruch 3, wobei das genannte Fluid durch den genannten Port injiziert wird und eines von Folgendem beinhaltet: ein Medikament; ein Fluid, das zur Verbesserung der anatomischen Bildgebung verwendet wird; fluoroskopischer Kontrastfarbstoff; Barium; ein radioaktives Material; Blut; Plasma; Kochsalzlösung; ein Blutbestandteil; eine Partikelsuspension; ein Nanogerät; und ein Nanomaterial.

5. Das Implantat-Abgabesystem nach Anspruch 3, wobei der genannte mindestens eine Fluid-Port (230) ferner konfiguriert ist, um mit einer außerhalb des genannten Körpers befindlichen Vorrichtung operativ gekoppelt zu werden.

6. Das Implantat-Abgabesystem nach Anspruch 5, wobei die genannte Vorrichtung ein Druckwandler ist, der konfiguriert ist, um einen Fluiddruck am distalen Ende der genannten ersten oder der genannten zweiten Hülle (204) zu messen.

7. Das Implantat-Abgabesystem nach Anspruch 6, wobei die genannte Messung des Fluiddrucks am distalen Ende der genannten ersten oder der genannten zweiten Hülle (204) verwendet wird, um die Genauigkeit des genannten Implantats (101) zu kalibrieren oder zu bewerten.

8. Das Implantat-Abgabesystem nach Anspruch 1, wobei die genannten Marker (205, 998) ein strahlenundurchlässiges Material beinhalten, das an mindestens einem von folgenden positioniert ist: einer distalen Spitze (201) der genannten ersten Hülle (202); dem distalen Abschnitt der genannten zweiten Hülle (204); einem Abschnitt des genannten Implantats (101); und als eine Vielzahl von Marker, die entlang eines Abschnitts der genannten ersten oder der genannten zweiten Hülle (202, 204) beabstandet sind.

9. Das Implantat-Abgabesystem nach Anspruch 1, wobei die genannten Marker (205, 998) an mindestens einem Anker (102, 103) am Implantat (101) angebracht sind.

10. Das Implantat-Abgabesystem nach Anspruch 1, wobei:
die genannte zweite Hülle (204) konfiguriert ist, um das Einführen einer Kathetervorrichtung zu ermöglichen, die so konfiguriert ist, dass sie selektiv am genannten Implantat (101) angebracht wird und das Implantat (101) proximal bewegt, wenn sie zurückgezogen wird;
wobei mindestens ein Anker (102, 103) des genannten Implantats (101) zusammenlegbar bzw. -klappbar ist (*collapsible*); und
wobei der genannte Anker (102, 103) des genannten Implantats (101) konfiguriert ist, um in einem zusammengeklappten Zustand platziert zu werden, wenn die genannte Kathetervorrichtung das Implantat (101) in Proximalrichtung in die genannte zweite Hülle (204) bewegt.

11. Das Implantat-Abgabesystem nach Anspruch 1, wobei die genannte zweite Hülle (204) ferner so konfiguriert ist, dass die genannte erste Hülle (202) in die genannte zweite Hülle (204) zurückgezogen werden kann, während das genannte Implantat (101) noch mit der genannten ersten Hülle (202) verbunden ist, und ferner so konfiguriert ist, dass sie die genannte erste Hülle (202) und das genannte Implantat (101) bedeckt, während das genannte Abgabesystem zurückgezogen und aus dem genannten Körper entfernt wird.

12. Das Implantat-Abgabesystem nach Anspruch 1, wobei das genannte Implantat (101) ferner ein starres Gehäuse und mindestens einen zusammenklappbaren Implantatanker (102, 103) umfasst, wobei der genannte mindestens eine Anker (102, 103) am genannten Gehäuse angebracht ist.

13. Das Implantat-Abgabesystem nach Anspruch 12, wobei der genannte Anker (102, 103) mindestens einen Draht umfasst, der durch mindestens ein Loch (124, 126) verläuft, das sich durch die Dicke des genannten Gehäuses erstreckt; und wobei ein erweiterter Abschnitt des Drahtes entlang des genannten Drahtes angeordnet ist, um den Draht im Loch (124, 126) zu halten; und
wobei das genannte Loch (124, 126) mit einem Senkbohrer bearbeitet bzw. versenkt ist, wobei das genannte versenkte Loch ein Loch (124) einer ersten Größe und ein Loch (126) einer zweiten Größe umfasst, wobei die erste Größe geringer ist als die zweite Größe, wobei das zweite Loch (126) nur teilweise durch das genannte Gehäuse verläuft, und wobei ferner der genannte erweiterte Abschnitt so konfiguriert ist, dass er in das genannte zweite Loch (126) passt.

14. Das Implantat-Abgabesystem nach Anspruch 13, wobei eine Vielzahl der genannten Marker in bekannten Abständen entlang des genannten Ankerdrahts angeordnet sind, um die Abschätzung der Entfernungen während einer angiographischen Bildgebung zu erleichtern; und wobei sich der genannte Marker auf dem Ankerdraht in der Nähe eines Punkts befindet, an dem der genannte Ankerdraht am genannten Gehäuse angebracht ist.

## Revendications

1. Un système de pose d'implant (200) comprenant :
un implant (101) incluant une surface tournée vers l'intérieur (132) et une surface tournée vers l'extérieur (142) opposée ;
une première gaine (202) et une deuxième gaine (204) s'étendant chacune à partir d'une extrémité proximale dudit système de pose d'implant (200), sachant qu'au moins ladite première gaine (202) s'étend jusqu'à une extrémité distale dudit système de pose d'implant (200), sachant que ladite première gaine (202) est positionnée au moins partiellement à l'intérieur de ladite deuxième gaine (204), la première gaine (202) pouvant être translatée par rapport à ladite deuxième gaine (204) ;
sachant que ledit implant (101) est relié à une surface extérieure de ladite première gaine (202), et
sachant que ladite première gaine (202) et ladite deuxième gaine (204) sont mobiles l'une par rapport à l'autre pour déployer ledit implant (101) vers un site cible dans une anatomie ;
**caractérisé par** :
ledit implant (101) comprenant en outre : une pluralité de marqueurs (205, 998) configurés pour être visibles avec un fluoroscope, sachant que la pluralité de marqueurs (205, 998) est positionnée le long dudit implant (101) selon un schéma asymétrique, que ledit schéma est configuré pour faciliter la détermination de l'orientation de l'implant lorsqu'il est visualisé sur un fluoroscope afin de déterminer laquelle de la surface tournée vers l'intérieur (132) ou de la surface tournée vers l'extérieur (142) opposée peut être visualisée, et que ledit schéma asymétrique comprend les marqueurs (205, 998) à trois des quatre coins d'un rectangle bidimensionnel lorsqu'il est visualisé normalement au plan dudit rectangle sur un fluoroscope.

2. Le système de pose d'implant d'après la revendication 1, sachant que ledit système de pose est configuré pour être partiellement inséré dans un vaisseau sanguin d'un corps humain de manière que ladite extrémité proximale reste externe audit corps et que ladite extrémité distale soit interne audit corps.

3. Le système de pose d'implant d'après la revendication 1, comprenant en outre au moins un port fluidique (230) positionné le long d'une extrémité proximale de ladite première gaine (202) ou deuxième gaine (204), ledit port fluidique (230) étant couplé fluidiquement à au moins une lumière (*lumen*) s'étendant sur la longueur de ladite première gaine (202) ou deuxième gaine (204) pour permettre l'écoulement de fluide à travers ladite lumière.

4. Le système de pose d'implant d'après la revendication 3, sachant que ledit fluide est injecté à travers ledit port et inclut l'un des éléments suivants : un médicament ; un fluide utilisé pour améliorer l'imagerie anatomique ; un colorant de contraste fluoroscopique ; du baryum ; une matière radioactive ; du sang ; du plasma ; une solution saline ; un composant sanguin ; une suspension de particules ; un nanodispositif ; et un nanomatériau.

5. Le système de pose d'implant d'après la revendication 3, sachant que ledit au moins un port fluidique (230) est en outre configuré pour se coupler de manière opérationnelle à un dispositif situé à l'extérieur dudit corps.

6. Le système de pose d'implant d'après la revendication 5, sachant que ledit dispositif est un transducteur de pression, configuré pour mesurer une pression de fluide à l'extrémité distale de ladite première ou de ladite deuxième gaine (204).

7. Le système de pose d'implant d'après la revendication 6, sachant que ladite mesure de la pression de fluide à l'extrémité distale de ladite première ou deuxième gaine (204) est utilisée pour calibrer ou évaluer la précision dudit implant (101).

8. Le système de pose d'implant d'après la revendication 1, sachant que lesdits marqueurs (205, 998) incluent un matériau radio-opaque positionné sur au moins l'un des éléments suivants : l'extrémité distale (201) de la première gaine (202) ; la partie distale de ladite deuxième gaine (204) ; une portion dudit implant (101) ; et sous la forme d'une pluralité de marqueurs espacées le long d'une portion de ladite première ou de ladite deuxième gaine (202, 204).

9. Le système de pose d'implant d'après la revendication 1, sachant que lesdits marqueurs (205, 998) sont attachés à au moins un dispositif d'ancrage (102, 103) sur l'implant (101).

10. Le système de pose d'implant d'après la revendication 1, sachant que :
ladite deuxième gaine (204) est configurée pour permettre l'insertion d'un dispositif cathéter configuré pour se fixer sélectivement à l'implant (101) et déplacer l'implant (101) de manière proximale lorsqu'il est rétracté ;
sachant qu'au moins un dispositif d'ancrage (102, 103) dudit implant (101) est repliable ; et
sachant que ledit dispositif d'ancrage (102, 103) dudit implant (101) est configuré pour être placé dans un état replié lorsque ledit dispositif cathéter déplace l'implant (101) de manière proximale dans ladite deuxième gaine (204).

11. Le système de pose d'implant d'après la revendication 1, sachant que ladite deuxième gaine (204) est en outre configurée pour permettre à ladite première gaine (202) d'être rétractée dans ladite deuxième gaine (204) alors que ledit implant (101) est toujours connecté à ladite première gaine (202), et en outre configurée pour recouvrir ladite première gaine (202) et ledit implant (101) alors que ledit système de pose est rétracté et retiré dudit corps.

12. Le système de pose d'implant d'après la revendication 1, sachant que ledit implant (101) comprend en outre un boîtier rigide, et au moins un dispositif d'ancrage d'implant repliable (102, 103), ledit au moins un dispositif d'ancrage (102, 103) étant fixé audit boîtier.

13. Le système de pose d'implant d'après la revendication 12, sachant que ledit dispositif d'ancrage (102, 103) comprend au moins un fil qui passe à travers au moins un trou (124, 126) qui s'étend à travers l'épaisseur dudit boîtier ; et qu'une portion élargie du fil est placée le long dudit fil pour retenir le fil dans le trou (124, 126) ; et
sachant que ledit trou (124, 126) est lamé, ledit trou lamé comprenant un trou de première taille (124) et un trou de deuxième taille (126), sachant que la première taille est inférieure à la deuxième taille, sachant que le deuxième trou (126) ne s'étend que partiellement à travers ledit boîtier, et en outre sachant que ladite portion élargie est configurée pour s'insérer dans ledit deuxième trou (126).

14. Le système de pose d'implant d'après la revendication 13, sachant qu'une pluralité desdits marqueurs sont espacés à des intervalles connus le long dudit fil d'ancrage pour faciliter l'estimation des distances pendant l'imagerie angiographique ; et sachant que ledit marqueur est situé sur le fil d'ancrage près d'un point où ledit fil d'ancrage s'attache audit boîtier.
